(19)

Europäisches
Patentamt
European
Patent Office
Office européen
des brevets

(11) **EP 4 439 064 A1**

(12) **EUROPEAN PATENT APPLICATION**

(43) Date of publication:
**02.10.2024 Bulletin 2024/40**

(21) Application number: **23165274.4**

(22) Date of filing: **29.03.2023**

(51) International Patent Classification (IPC):
**G01N 33/542** (2006.01)     **G01N 15/14** (2024.01)
**G01N 21/64** (2006.01)

(52) Cooperative Patent Classification (CPC):
**G01N 15/1431; G01N 15/1429; G01N 15/1433;
G01N 21/6458; G01N 33/542;** G01N 2015/1006;
G01N 2015/1445; G01N 2015/1488

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Stamou, Dimitrios
2920 Charlottenlund (DK)**

(72) Inventors:
• **STAMOU, Dimitrios
2920 Charlottenlund (DK)**

• **KOCKELKOREN, Gabriele
94107 San Francisco (US)**
• **LAURITSEN, Line
5230 Odense M (DK)**
• **SHUTTLE, Christopher
00870 Helsinki (FI)**
• **BREUER, Artu
2100 Østerbro, Copenhagen (DK)**
• **KAZEPIDOU, Eleftheria
2400 Copenhagen (DK)**

(74) Representative: **Schreiter, Christoph
Schreiter-IP
Staudenrauchstrasse 19
80939 München (DE)**

(54) **SPATIALLY RESOLVED MEMBRANE MOLECULE ANALYSIS**

(57) The present invention suggests a method and a system for characterizing a membrane bound molecule. The method comprises determining a plurality of spatially resolved membrane properties of a biological membrane, determining, for a plurality of locations, a spatially resolved membrane molecule signal related to the membrane bound molecule at the plurality of locations on the membrane and grouping the spatially resolved membrane molecule signals to classes of the membrane properties to obtain a first state specific membrane molecule pattern. The method further comprises inducing a conformational change to the membrane bound molecule, and monitoring a change in the grouping of the spatially resolved receptor signals and obtaining a second state specific membrane molecule pattern.

Fig. 1

EP 4 439 064 A1

**Description**

The present invention relates to methods, apparatuses and systems for analysing biological molecules and their pharmacological parameters in cell membranes. In particular, the present disclosure relates methods, systems, and apparatuses for fluorescence imaging of conformational states and changes in the conformational states of membrane bound molecules.

Many biological molecules can occur in different conformational states and can change the conformational state upon external triggers. These conformational states and conformational changes underpin the function of biological molecules and are thus important information for many biological and pharmacological processes. For example, many methods were developed to determine ligand binding to receptor proteins and/or conformational changes of proteins in cell membranes. Among others, fluorescence methods and techniques are used today to determine the efficacy of ligand binding and to monitor conformational changes in the biomolecules.

Most methods used today determine conformational changes in bulk thus averaging over many molecules in a single cell and typically many cells in a cell culture. Some methods have been developed to analyse ligand binding or conformational changes on a single molecule level but most industrial applications measure ligand binding and conformational changes of biomolecules in bulk.

Membrane molecules are complex biological systems and there are many additional effects that cannot be revealed by the methods available today.

Recently developed methods have shown that membrane molecules form clusters in specific areas of cell membranes.

There is a need for improved apparatuses and methods for measuring conformational states and/or conformational changes of biomolecules.

Summary of the invention

The present invention suggests a method and a system for characterizing a membrane bound molecule. The method comprises determining a plurality of spatially resolved membrane properties of a biological membrane, determining, for a plurality of locations, a spatially resolved membrane molecule signal related to the membrane bound molecule at the plurality of locations on the membrane and grouping the spatially resolved membrane molecule signals to classes of the membrane properties to obtain a first state specific membrane molecule pattern. The method further comprises inducing a conformational change to the membrane bound molecule, and monitoring a change in the grouping of the spatially resolved receptor signals and obtaining a second state specific membrane molecule pattern.

In a second aspect, the present invention discloses a method for characterizing a state of a membrane bound molecule. The method comprises determining a plurality of spatially resolved membrane properties of a biological membrane, determining, for a plurality of locations, a spatially resolved membrane molecule signal related to the membrane bound molecule at the plurality of locations on the membrane, and grouping the spatially resolved membrane molecule signals to classes of the membrane properties to obtain a state specific membrane molecule pattern. The method further comprises comparing the state specific membrane molecule pattern with patterns in a database and identifying a conformational state of the membrane bound molecule.

The methods are alternative solutions that relate to the identification and characterization of state specific patterns on one end and to the identification of specific states based on the pre-characterized state specific patterns.

The present disclosure also suggests an apparatus and a system for carrying out the methods of the present disclosure.

Description of the figures

The invention may be better understood when reading the detailed description of examples of the present disclosure which is given with respect to the accompanying figures in which:

Fig. 1 shows the generation of high-accuracy topography maps of plasma membranes of living cells,
Fig. 2 shows an example of how receptor density and activation is organized into domains at the plasma membrane,
Fig. 3 shows an example of domain-based characterization of different ligands and different concentrations of the same ligand,
Fig. 4 shows examples of conformational states of GLP1R are segregated by plasma membrane curvature, and
Fig. 5 shows examples of how different ligands give rise to different patterns of beta1 AR activation in curvature space.

Detailed description

Examples of the present disclosure will now be described in more detail. It is to be understood that the described examples and the examples shown in the figures are purely illustrative for the methods described. The methods of the present disclosure are not limited to specific biological systems and a person skilled in the art will amend the examples according to specific requirements. It is not necessary to implement all features shown in the examples and a person skilled in the art will combine features shown with respect to one figure with examples shown in other figures.

The present disclosure relates to methods, systems and apparatus' for characterizing or determining at least one parameter and/or states of membrane molecules. Membrane molecules are molecules that are linked to, attached to or integrated in a membrane and are termed membraned bound molecules herein. A group of membrane bound molecules of interest for medical and pharmacological applications are membrane proteins such as membrane bound proteins that are attached to a biological membrane or transmembrane proteins that extend through a biological membrane.

Membranes are biological membranes made from a plurality of lipid molecules in the examples. Biological membranes are typically lipid bilayers and may be termed lipid membranes. Examples for the biological membrane are cell membranes, in particular the plasma membrane of a cell. Membrane molecules also comprise lipids or groups of lipids. So further examples refer to biological membranes. However, the concept of the present disclosure may be applied to other membranes systems in a similar manner.

Among membrane proteins, ligand receptors are a group of membrane bound proteins that are of pharmacological interest. Examples of the present disclosure were carried with G-protein coupled receptors (GPCRs) and receptor tyrosine kinases as examples systems. The method of the present disclosure has been tested with these systems but the methods are generic. It is evident that the methods disclosed herein are not limited by the specific receptors used in the examples. The methods can be applied with other receptors or other membrane molecules in a corresponding way. Examples for other membrane molecules comprise, but are not limited to ion channels, enzymes or transporters.

In a first aspect, the method comprises determining a plurality of spatially resolved membrane properties of the biological membrane, determining, for a plurality of locations, a spatially resolved membrane molecule signal related to the membrane bound molecule at the plurality of locations on the membrane, and grouping the spatially resolved membrane molecule signals to classes of the membrane properties to obtain a first state specific membrane molecule pattern.

Determining the plurality of spatially resolved membrane properties comprises determining the membrane property in different areas of the membrane. In other words, determining the plurality of spatially resolved membrane properties comprises determining membrane property values representative for the membrane property in different distinct membrane areas. A membrane area can be a certain area or section on the membrane, like a pixel. The membrane area may also be a voxel, as the membrane has a three dimensional position in space. The membrane properties may be recorded in two-dimensional matrix and/or may be plotted in a two-dimensional map.

The size of the membrane area, and such the spatial resolution of the spatially resolved membrane properties can be chosen depending on the membrane and the technology used. If fluorescent labels are used, a resolution of 250 nm has a sufficient disclosure. 10 nm or better can be achieved. An area can correspond to the resolution of the measurement set-up or can be chosen differently.

The membrane properties may be a topography, a curvature, a tension or a fluidity of the membrane or any combination thereof. In another example the membrane property may be a presence or absence of specific membrane components. In yet another example the membrane property may be the density of a membrane lipid molecule and/or the lipid composition of the membrane. Other membrane properties or a combination of membrane properties may also be used. The membrane property may be an average or mean value in each of the areas, pixels or voxels. The membrane property may be determined in a spatially resolved manner by microscopy. Microscopy may be implemented using light, for example transmitted, reflected, fluorescent or bioluminescent, or electrons. Other imaging mechanisms or principles may also be used to acquire microscopy images.

Determining, for a plurality of locations, a spatially resolved membrane molecule signal related to the membrane bound molecule comprises determining a spatial position of the signal related to the membrane. Spatial resolution of the density may vary on the requirements and may be adapted to the biological system under investigation. It may correspond to the spatial resolution of the membrane properties but a different resolution may be used as well.

Determining, for a plurality of locations, a spatially resolved membrane molecule signal may comprise fluorescent imaging of fluorescent labels that can represent the presence of the membrane molecule. For example, the fluorescent label may be attached to the membrane molecule. Examples for fluorescent label include, but are not limited to, fluorescent proteins or organic dyes.

Fluorescent imaging may comprise confocal microscopy. Alternative methods are wide field, TIRF, spinning disk confocal, lightsheet, STED, PALM, STORM and other methods that allow to determine a density of fluorescent signals on the biological membrane.

Classes, categories of groups of membrane properties may be defined. For example, a multi-dimensional matrix and/or map of the membrane properties may be determined to provide a spatially resolved map in the dimensions of the membrane properties. The map may be two dimensional or may comprise more dimensions. Areas of the membrane may be categorized according to the membrane properties. For example, membrane areas with a membrane mean curvature larger than a pre-determined value may be classified as area with high mean curvature and other areas may be classified as areas with low mean curvature. Similar areas can be classified depending in the two principal curvatures, lipid density, protein density or on any other membrane property of interest. In some examples domains where a membrane property exceeds a pre-determined value may be identified.

The membrane properties have a local influence on the behaviour or characteristics of the membrane protein. The protein signal may be different in areas of higher membrane curvature compared to areas that have no or only a small membrane curvature. Other examples revealed, that the protein signal varies depending on at least one of a presence of specific lipids in the membrane, a lipid composition, a density of lipids.

The spatially resolved membrane molecule signals are grouped into the classes of the membrane properties to obtain a first state specific membrane molecule pattern. The classes of membrane properties are described above. For example, the signals may be grouped in a membrane curvature space resolving the membrane principal curvatures $C1$ and $C2$ in two dimensions. Besides membrane curvature space, a lipid density space or a lipid composition space may be used.

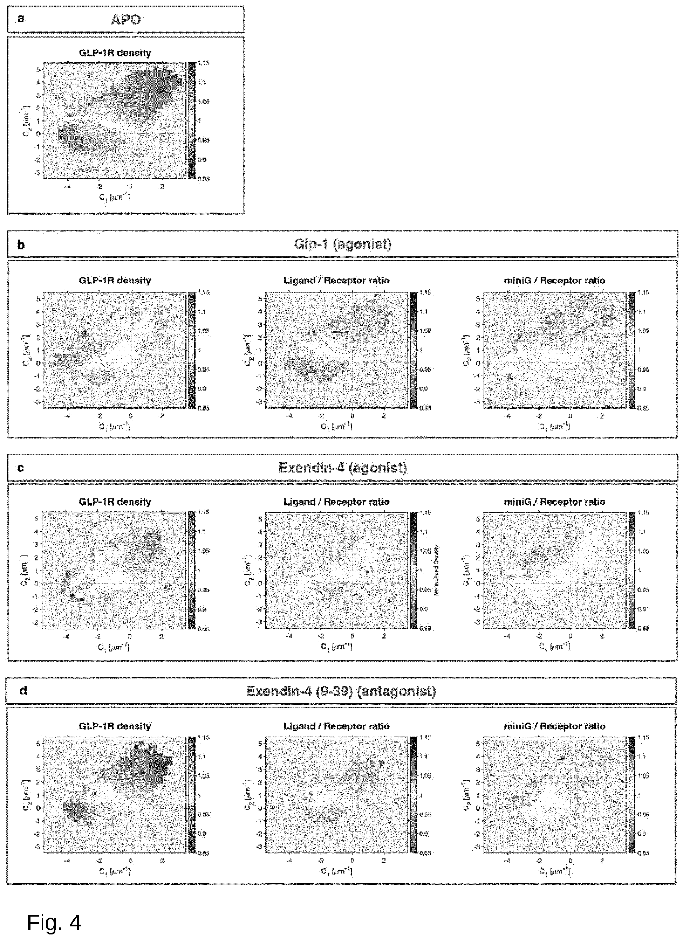

The grouping of the spatially resolved membrane molecule signals results in a specific pattern that is specific for a membrane molecule in a particular state and a particular membrane. The pattern may be a one-, two-, or multidimensional representation of the grouped or classified membrane molecules signals. The pattern may have domains, maxima, minima and other features that a representative for a graph or map. An example for such a pattern in the curvature space is shown in Figure 4a. Other examples may be lipid density or lipid composition. The representation may then be one dimensional or may be multidimensional in case a multi-lipid composition. The particular state may correspond to a conformational state of the membrane molecule.

The inventors found that this pattern changes in dependence of the environment of the membrane molecule and/or in dependence of a conformational state of the membrane molecule. For example, the pattern changes if ligands are added to the receptors. Moreover, the pattern changes specifically depending on the ligand, on the type of ligand and on the ligand concentration. Thus, a first pattern corresponds to a first conformational change and, a second pattern corresponds to a second conformational change.

In one aspect, this allows to identify specific patterns, that are representative for a conformational state of the membrane molecule. Thus, the method comprises one aspect to induce a conformational change to the membrane bound molecule, and monitoring a change in the grouping of the spatially resolved receptor signals and obtaining a second state specific membrane molecule pattern. Thus, the second state specific membrane molecule pattern is specific to the induced change. For example, the addition of a particular ligand at a particular concentration will induce a pattern that is specific for this situation. The induced conformation change, can be the addition of ligands, agonist, antagonist. It can also be another environmental change, such as temperature, pH-value or others.

The method allows to determine and characterize conformational changes of the membrane molecule itself or along a signal pathway in dependence of the molecule density at or in the biological membrane.

In some examples, the determining the spatially resolved membrane molecule signal comprises determining at least one of a ratio of signals of an antibody to membrane bound molecule, ligand to antibody and ligand to membrane bound molecule. This provides more detailed insight in the conformation states of some of the membrane molecules.

In a second aspect, the specific patterns and/or matrices may be registered in a look-up table. This look-up table can be a database. The data base may then contain a plurality of patterns that are specific and representative for a state of the membrane molecules. A pattern measured as described above can then be compared to patterns from the look-up table to identify a conformational state of the membrane molecule.

In yet other aspects, the method comprises determining a spatial molecule density related to a density of the membrane molecule for a plurality of locations on the biological membrane. The method further comprises determining a conformational change of the membrane protein related to at least one of the plurality of locations, and correlating the conformational change to the spatial molecule density of the membrane molecule.

In one example, fluorescence may be used for determining the conformational change relate to the membrane molecule. A second type of fluorescent labels may be attached to the molecule or in the signalling pathway of the membrane molecule, where the fluorescens properties alter in dependence of the conformational change. Fluorescence methods with different fluorescent tags can be used with substantially the same measurements system, where for example different wavelength for different fluorescent tags are used. However, other ways to determine conformational changes have been developed in recent years that can be used with the present disclosure as well requiring additional measurement set-up.

Correlating the conformational change to the spatial molecule density of the membrane molecule comprises in one example evaluating conformational changes in dependence of the molecule density. The inventors have found the conformational changes occur differently depending on the molecule density. In an example, membrane molecules are grouped in two or more classes of densities and the conformational changes are determined for each of the classes. For example, the response to ligand binding, may be different depending on the molecule density. Density dependent ligand binding or ligand efficacy may be determined in one example. In another example, the change of the conformational state of membrane molecule, for example a membrane protein may depend on the density of the membrane molecules. One state may be favourable over a second state in areas with higher or lower membrane molecule density.

3D measurements reveal information of receptor activation and localization in real space (x, y and z) and in curvature space. This allows for the pharmacological characterization of ligands in both parameter spaces.

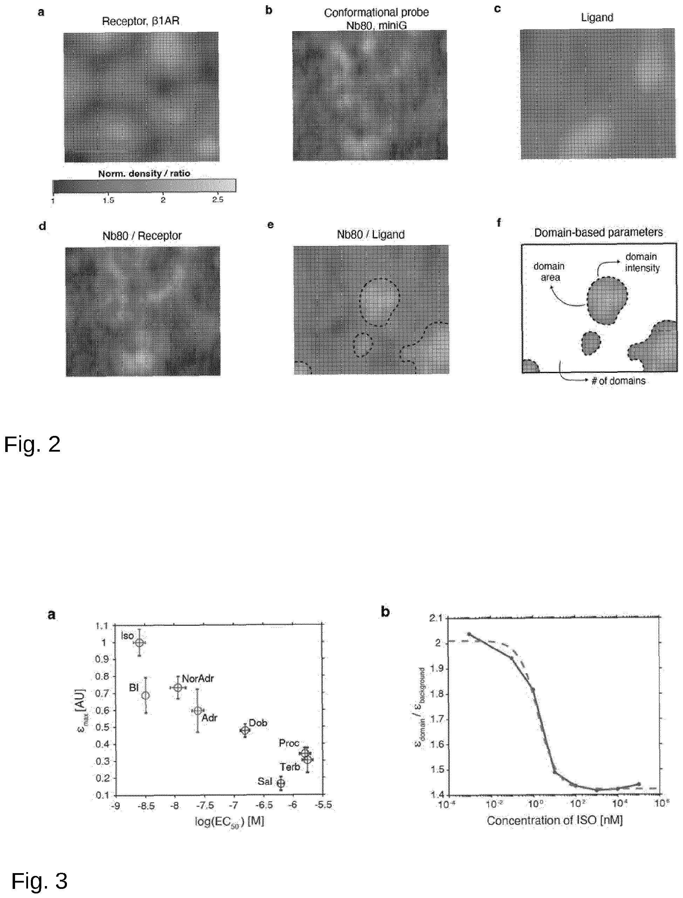

The methods and systems allow to obtain the relative density of receptors, conformational probes, and ligands (See, Figure 2a-c as examples) mapped onto the 3D surface of the plasma membrane of living cells. The inventors found that these three key signalling components are spatially organized into domains.

These local density measurements are used to gain understanding of spatially resolved receptor signalling. In one aspect, the ratio of the conformational probe and the receptor density are mapped as a measure for the relative activation probability (Seen Figure 2d as an example). Second, the intrinsic efficacy of ligands can be spatially resolved by mapping the ratio of the conformational probe to ligand density (Exemplary results shown in in Figure 2e).

The probability for a ligand to activate a receptor and, subsequently stabilize the conformation is organized into domains at the plasma membrane as can be seen from the examples of Figs. 2d and 2e. By applying a thresholding algorithm to isolate individual domains, a ligand can be characterized by its intrinsic efficacy, by the area of individual domains and by the number of domains it creates (Figure 2f). This methodology can be employed to characterize any ligand for any ligand concentration of interest and is not limited to the examples shown and described.

The methods and systems described herein have been evaluated and tested in several experiments using several exemplary systems. Some examples are described below. It is obvious to a person skilled in the art that the methods, systems, and corresponding apparatuses are not limited to these biological examples and that the method and systems, and apparatuses can be used with other membrane bound systems.

Examples

Live-cell microscopy

Imaging was performed on an Abberior Expert Line system with an Olympus IX83 microscope (Abberior Instruments GmbH). For imaging SNAP-Surface649 and eGFP we used respectively 640 nm or 488 nm pulsed excitation laser; fluorescence was detected between 650-720 nm or 500-550 nm, respectively. For imaging mRuby2 and HALO-JF549 we used 561 nm pulsed excitation and fluorescence was detected between 580 - 630 nm. Cross-excitation was avoided by sequential imaging. All XZY stacks were recorded by piezostage (P-736 Pinano, Physik Instrumente, Germany) scanning using a voxel size of 30 nm (dx=dy=dz=30 nm). We used a UPlanSApo x100/1.40 oil immersion objective lens and a pinhole size of 1.0 Airy units (i.e., 100 $\boxed{?}$ m). Alignment of confocal channels was adjusted and verified on Abberior auto-alignment sample. All measurements were made at room temperature and acquired in confocal imaging mode.

Data Analysis

The 3D imaging method of the present disclosure simultaneously, but independently, recovers 1) high-accuracy membrane topography and curvature and 2) protein density of any membrane-associated protein of interest. In the sections below, these two key principles are described in detail and considerations in method development are outlined.

Reconstructing high-accuracy topography maps with confocal microscopy

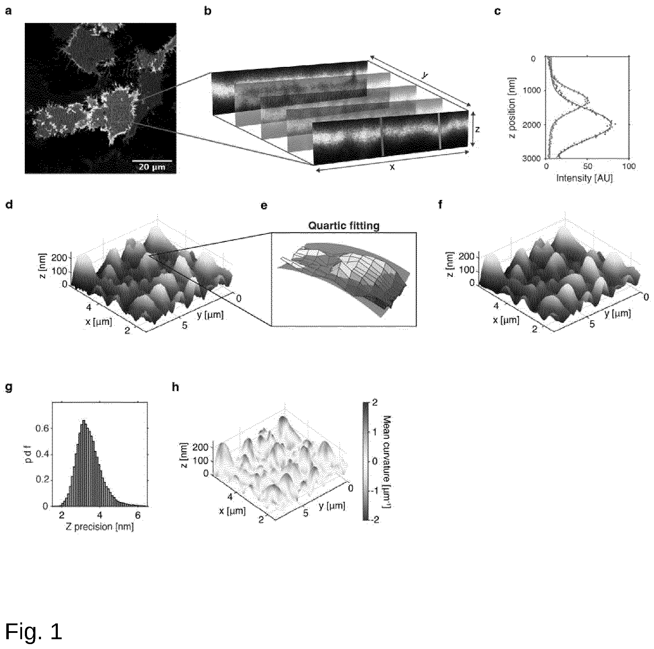

The 3D imaging method obtains the Z position of the adherent part of the plasma membrane of living cells with high accuracy (Figure 1). We image the plasma membrane in three dimensions by XZY stacks with a voxel size of 30 nm. Examples are shown in Figures 1a and 1b. For each XY-pixel we extract an intensity profile in Z, which is fitted with a Gaussian function (Example in Figure 1c). Here, the fit to the data provides a good estimation of the Z position of the membrane.

Hereafter, a topography map of the surface is generated from the Z positions directly obtained by the Gaussian fits, as seen at an example in Figure 1d. To improve the localization accuracy, a denoising approach was deployed that removes the high-frequency noise, while maintaining fine spatial fluctuations in a supervised manner. The topography map was treated as a noisy point cloud and error weighted quartic fits are used to retrieve a high accuracy estimate of the Z position and principal curvatures of each pixel4. The surface is fitted pixelwise with a quartic fit (Eq. 1, see Methods) with a window size that is related to the diffraction limit in XY. As a result, a denoised surface was recovered with a mean accuracy in Z of 3.1±1 nm, which reflect the errors associated with the pixelwise estimation of the Z position.

Next, the mean and Gaussian curvature of the recovered surface is calculated using their analytical expressions (Eq. 2 and 3, see Methods). Using the mean and Gaussian curvatures, also the two principal curvatures (Eq. 4, see Methods) were calculated. The two principal curvatures give a measure of the maximum and minimum bending of each point and represent the overall geometry of a point.

Reconstructing high-accuracy topography maps 3D imaging

3D membrane topography was reconstructed by a software implemented method. Briefly, XY-slices were smoothened with a mean filter of 3x3 pixels and every XY-position was fitted with a Gaussian in the Z-direction (Figure 1c). The Z position of the peak of the Gaussian fit localized the Z-position of the plasma membrane (Figure 1c, ref.5). The amplitude, i.e. maximum intensity, of the Gaussian fit is proportional to the density of the protein in each pixel.

Poor fits were filtered out using the error metrics from the Gaussian fits based on R2 and uncertainties of the Z position and maximum intensity. Additionally, to remove non-diffraction limited membrane structures, fitted data where the full width at half maximum (FWHM) of the Gaussian significantly exceeds the diffraction limit in Z was removed.

Using dx=dy=30 nm as pixel size in this example, quartic fits were used to denoise the surface extracted by the Z position of Gaussian fits. Each pixel, surrounded by a neighboring pixel window related to the resolution in XY, was fitted to Eq. 1:

$$f(x,y) = a1 \cdot x^2 + a2 \cdot x \cdot y + a3 \cdot y^2 + a4 \cdot x + a5 \cdot y + a6 \tag{1}$$

where a1-a6 are constants. A major advantage of fitting the surface to Eq. 1, is that it provides the ability to obtain an analytical expression for the mean (Eq. 2) and the Gaussian curvature (Eq. 3) of each pixel, H and K respectively.

$$H = ((1+f_y^2) \cdot f_{xx} - 2 \cdot f_x \cdot f_y \cdot f_{xy} + (1+f_x^2) f_{yy}) / (2 \cdot (1+f_x^2+f_y^2)^{3/2}) \tag{2}$$

$$K = (f_{xx} \cdot f_{yy} - f_{xy}^2) / (1+f_x^2+f_y^2)^2 \tag{3}$$

Here, the functions are defined as first and second order derivatives of Eq. 1:
$f_x=2 \cdot a1x+a2y+a4$, $f_y=a2x+2 \cdot a3y+a5$, $f_{xx}=2 \cdot a1$, $f_{yy}=2 \cdot a3$, and $f_{xy}=a2$.

The quartic fit of each pixel is error weighted by the error associated with the determination of the Z position from the Gaussian profile fits. The error weighted mean average of the quartic fitted surfaces for a 3x3 grid is taken for the Z position, mean and Gaussian curvatures. By using dx=dy=30 nm, the 3x3 pixels will correspond to an area of 90x90 nm, which is a factor of two below the resolution limit. These 9 pixels can be considered as an independent technical repeat measurement, thus an error weighted standard error of the mean can be used for estimating the accuracy of the Z position. This results in a high precision topography map of the adherent cell membrane of a living cell.

Recovering receptor density from Gaussian fits

This approach makes use of Gaussian fits to recover the position of the membrane in Z (Z-location of the peak of the Gaussian curve, see previous section) and the density of protein (the maximum intensity of the Gaussian curve). The maximum intensity of the Gaussian profile depends on the total amount of labelled receptors and the membrane area that is passing through the confocal volume of the point we are sampling. The latter will vary depending on the angle of the membrane that crosses the confocal volume. This example normalizes for this variation in membrane angles by dividing the maximum intensity of the Gaussian fit by the membrane area crossing the sampled confocal volume. This results in the most accurate representation of receptor density on the recovered topography maps.

Filtering criteria for Gaussian fits

Several filtering criteria were imposed to improve the accuracy of the recovered topography surface and protein density. First, fits with an adjusted R-squared below 0.9 are removed from further analysis. Second, the standard error of the fit for the maximum intensity of the Gaussian must be smaller than 30% of the value of maximum intensity. Third, the standard error of the fit for the Z position should be smaller than 100 nm. Fourth, we filter out Gaussian fits with full width at half maxima (FWHM) larger than 800 nm and smaller than 600 nm. This filter allows us to remove any membrane features that are larger than the axial diffraction limit and do not correspond to a simple membrane bilayer. Collectively, the above filtering accepts typically ~ 80 % of gaussian fits.

Recovering ligand density

Density of fluorescently labelled objects, such as ligands, antibodies has been calculated by averaging along the optical axis the intensity of the ligand channel measured 4 pixels above and below the recovered Z position. This density has been corrected for background signal.

Example 1

Cell lines

Human embryonic kidney (HEK293) cells (ATCC®CRL-1573™) were cultured in DMEM supplemented with 10 % FBS. Cell lines were tested routinely for mycoplasma by Eurofins Genomics Mycoplasmacheck. All cell lines were grown at 37 °C, 5 % CO2, in an atmosphere with 100 % humidity.

Cell transfection

All cell lines were grown in 8-well Ibidi® chambers with glass bottoms.,. For each well, a solution of plasmid, Lipofectamine™ LTX Reagent with PLUS™ was made according to manufacturers' protocol in the ratio of 1:3:1, and OptiMEM was added to a final volume of 25 μL. The amount of plasmid used for each well was 0.25 μg β1AR-SNAP, 0.25 μg β2AR-SNAP and 0.25 μg GLP1R-SNAP. After transfection, the cells were left to grow for about 16 hours before imaging.

Activation of β1AR, β2AR and GLP1R is measured by recruitment of miniG$_s$, an engineered version of G protein ☐ s. Similarly, activation of β1AR and β2AR can be probed by nanobody 80 (Nb80), which specifically recognizes the active conformation of β1AR and P2AR. As described above, for each well, a solution of plasmid, Lipofectamine™ LTX Reagent with PLUS™ was made according to manufacturers' protocol in the ratio of 1:3:1, and OptiMEM was added to a final volume of 25 μL. The amount of plasmid used for each well was 0.25 μg β1AR-SNAP and 0.188 μg eGFP-miniGs or 0.188 μg HALO-miniGs or 0.188 μg mRuby2-miniGs or 0.188 μg of eGFP-Nb80, 0.25 μg β2AR-SNAP and 0.188 μg eGFP-miniGs or 0.188 μg of eGFP-Nb80, and 0.25 μg GLP1R-SNAP and 0.188 μg HALO-miniGs. After transfection, the cells were left to grow for about 16 hours before imaging.

Ligands

Activation of β1AR was measured for the ligands: isoproterenol hydrochloride, BI-167107 and BI-PEG-KK114, epinephrine hydrochloride, norepinephrine bitartrate salt, salbutamol hemisulfate salt, dobutamine hydrochloride, procaterol hydrochloride, CGP20712A methane sulfonate salt, atenolol, carvedilol and carazolol-KK114. Activation of GLP1R was measured for the ligands: [Leu14,Leu28]-Exendin4-Alexa488, [Aib8]-GLP-1(7-37)-Alexa488 and Exendin4[9-39]- Alexa488.

Description of the experiments and Measurement principle

Live-cell protein labelling and receptor activation

To selectively label PM GPCRs we took the following measures: 1) we tagged receptors on the extracellular N terminus and selectively labelled PM GPCRs using cell-impermeable SNAP technology1, 2) we imaged within ~10 min from fluorescent labelling and in the absence of agonists, to minimize the chance of constitutive and ligand mediated internalization2, 3) we validated the method with a prototypic GPCR that is known to reside mostly in the PM, the ☐ 1 adrenergic receptor (β1AR)[3].

Prior to imaging, SNAP-tagged β1AR, β2AR, GLP1R were labelled with SNAP649 according to manufacturers' protocol. Briefly, the cell medium was removed from each well and 100 μL of new medium premixed with 0.5 μL of a 50 nmol/μL solution of SNAP-Surface® was added to the cells and the labelling reaction proceeded for 10 min at 37 °C. Next, the medium was replaced with 200 μL of Leibovitz's medium and the sample was washed 3 times before imaging.

Results

Figure 1 shows the generation of high-accuracy topography maps of plasma membranes of living cells. Figure 1a shows a fluorescence confocal image of HEK293 cells over-expressing a SNAP construct of the β1AR receptor labeled with SNAPSurface649. Figure 2b illustrates an XZY-stack (dz/dx/dy = 30 nm) of the highlighted area in Figure 1a. Figure 1c shown extracted intensity Z-profiles of the blue and red linescans highlighted in Figure 1b and their corresponding Gaussian fits overlaid. The axial position of the Gaussian peak corresponds to the Z position of the membrane, whereas the amplitude of the Gaussian peak is proportional to protein density. Figure 1d is a topography map of the area shown in Figure 1b reconstructed from the Z positions obtained from the Gaussian fitting. Figure 1e is a local error weighted quartic fit for a 3x3 pixel, 90 nm x 90 nm, area using Eq. 1 (see Methods). Figure 1f is a recovered denoised topography map after quartic fitting (same area as in Figure 1d). Figure 1g shows the localization precision of the Z positions calculated as the error weighed standard error of the mean for a 3x3 pixel, 90 nm x 90 nm, area of the denoised topography maps. Because this 90x90 nm area is a factor of two below the resolution limit we consider these 9 pixels as an independent technical repeat measurement. Figure 1f is a topography map from Figure 1f overlaid with mean membrane curvature.

Figure 2 shows examples of how receptor density and activation is organized into domains at the plasma membrane. Figure 2a is 2D projection of normalized receptor density (β1AR). Figure 2b is a 2D projection of Nb80 density, a typical conformational probe used for the β1AR and β2AR receptor. Figure 2c shows a 2D projection of fluorescent ligand density, for BI-PEG-KK114. Figure 2d shows a 2D projection of ratio Nb80 / β1AR, i.e. conformational probe to receptor, reveals spatial variations in activation probability. Figure 2e shows a 2D projection for ratio Nb80 / BI-PEG-KK114, i.e. conformational probe to ligand, reveals spatial variations in intrinsic efficacy. Figure 2f shows a schematic of domain-based data analysis shows domains of high ratio above a defined threshold. Outlined are isolated domains. This allows us extract 3 ligand specific parameters: 1) the area of a domain, 2) the ratio in a domain and 3) the number of domains produced by a ligand.

Figure 3 shows exemplary a domain-based characterization of different ligands and different concentrations of the same ligand. Figure 3a is a scatter plot of the intrinsic efficacy of domains versus $\log(EC_{50})$ for 8 different ligands. Data points in green are full agonists and partial agonists are shown in purple. Errorbars in y represent standard error of the mean and in x represent the errors from ref. [6]. Figure 3b is the ratio of intrinsic efficacy in domains over intrinsic efficacy in background plotted against concentration of isoproterenol. Data points are shown in blue and red dashed line is a fit.

Figure 4 shows as an example of the method that conformational states of GLP1R are segregated by plasma membrane curvature. Figure 4a is a curvatures space plot (C1 vs. C2) of relative GLP1R density in the absence of ligand. Figures 4b to 4d are curvature space plots (C1 vs. C2) of relative GLP1R density (left panel), relative ligand / receptor ratio (middle panel) and relative miniGs / receptor (right panel) for the agonists Glp-1 (Fig. 4b) and Exendin-4 (Fig. 4c) and antagonist Exendin-4 (9-39) (Fig. 4d).

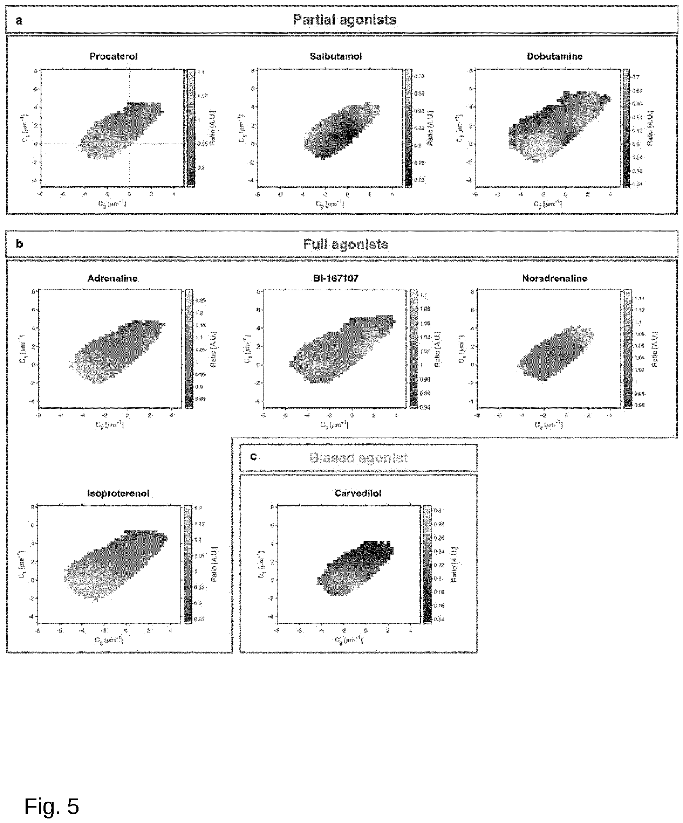

Figure 5 shows an example results where different β1AR ligands give rise to different patterns of activation in curvature space. Figures 5a to c are curvature space plots (C1 vs. C2) for Nb80 / β1AR ratio for 3 partial agonists (Figure 5a), 4 full agonists (Figure 5b) and 1 biased agonist (Figure 5c).

The methods and systems have been tested with different ligands. The methods and systems can be used to study spatial patterns of receptor activation for any receptor and any ligand at any ligand concentration. It is evident that the principles described are not limited to ligand-receptor interactions but that any other conformational change in a membrane bound molecule can be detected and characterized. The conformation change may be triggered by ligand interaction or any other change in the environment of the membrane bound molecule.

**Claims**

**1.** A method for characterizing a state of a membrane bound molecule, the method comprising:

> determining a plurality of spatially resolved membrane properties of a biological membrane,
> determining, for a plurality of locations, a spatially resolved membrane molecule signal related to the membrane bound molecule at the plurality of locations,
> grouping the spatially resolved membrane molecule signals to classes of the membrane properties to obtain a state specific membrane molecule pattern,
> comparing the state specific membrane molecule pattern with patterns in a database and identifying a conformational state of the membrane bound molecule.

**2.** A method for characterizing a membrane bound molecule, the method comprising:

determining a plurality of spatially resolved membrane properties of a biological membrane,

determining, for a plurality of locations, a spatially resolved membrane molecule signal related to the membrane bound molecule at the plurality of locations,

grouping the spatially resolved membrane molecule signals to classes of the membrane properties to obtain a first state specific membrane molecule pattern,

inducing a conformational change to the membrane bound molecule, and

monitoring a change in the grouping of the spatially resolved receptor signals and obtaining a second state specific membrane molecule pattern.

3. The method of claim 2, further comprising storing the second state specific membrane molecule pattern in a database representative for the conformational state induced by the conformational change.

4. The method of claim 2 or 3, wherein inducing a conformational change to the membrane bound molecule comprises addition of at least one of a ligand and an antibody.

5. The method of any one of the preceding claims, wherein the determining the spatially resolved membrane molecule signal comprises detecting a signal of at least one of a tag attached to the membrane molecule, an antibody to the membrane bound molecule, a ligand to the membrane bound molecule.

6. The method of any one of the preceding claims, wherein the determining the spatially resolved membrane molecule signal comprises determining at least one of a ratio of signals of an antibody to membrane bound molecule, ligand to antibody and ligand to membrane bound molecule.

7. The method of any one of the preceding claims, wherein the biological membrane is a plasma membrane of a biological cell.

8. The method of any one of the preceding claims, wherein the membrane molecule is a membrane bound receptor protein.

9. The method of any one of the preceding claims, wherein the plurality of spatially resolved membrane properties are topography parameters of the membrane.

10. The method of any one of the preceding claims, wherein the plurality of spatially resolved membrane properties comprise at least one membrane curvature (C1, C2).

11. The method of any one of the preceding claims, wherein determining the plurality of spatially resolved membrane properties comprises fluorescence imaging.

12. The method of any one of the preceding claims, wherein the determining, for a plurality of locations, the spatially resolved membrane molecule signal, comprises fluorescence imaging of fluorescent tags attached to at least one of the membrane molecule, a ligand to membrane molecule, and an antibody to the membrane molecule.

13. An apparatus for characterizing a membrane bound molecule, the apparatus comprising a fluorescence imaging device for imaging fluorescence in and at cell membranes and a data evaluation, the data evaluation implementing the methods of any of the preceding claim.

14. A data storage comprising data representative for a state specific membrane molecule pattern in a membrane property spaces and representative for the conformational state of the membrane molecule.

Fig. 1

**a** Receptor, β1AR

**b** Conformational probe Nb80, miniG

**c** Ligand

Norm. density / ratio

1    1.5    2    2.5

**d** Nb80 / Receptor

**e** Nb80 / Ligand

**f** Domain-based parameters

domain area

domain intensity

# of domains

Fig. 2

Fig. 3

Fig. 4

Fig. 5

Europäisches
Patentamt

European
Patent Office

Office européen
des brevets

# EUROPEAN SEARCH REPORT

Application Number

EP 23 16 5274

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ROSHOLM KADLA R ET AL: "Membrane curvature regulates ligand-specific membrane sorting of GPCRs in living cells", NATURE CHEMICAL BIOLOGY, vol. 13, no. 7, 8 May 2017 (2017-05-08), pages 724-729, XP093079837, New York ISSN: 1552-4450, DOI: 10.1038/nchembio.2372 Retrieved from the Internet: URL:http://www.nature.com/articles/nchembio.2372> * abstract * * page 724, column 2, paragraph 3 - page 725, column 2, paragraph 2 * * page 726, column 2, paragraph 2 - page 727, column 2, paragraph 1 * | 1-14 | INV. G01N33/542 G01N15/14 G01N21/64 |
| Y | SOUBIAS OLIVIER ET AL: "The role of membrane curvature elastic stress for function of rhodopsin-like G protein-coupled receptors", BIOCHIMIE, MASSON, PARIS, FR, vol. 107, 23 October 2014 (2014-10-23), pages 28-32, XP029105033, ISSN: 0300-9084, DOI: 10.1016/J.BIOCHI.2014.10.011 * the whole document * | 1-14 | |
| Y | US 2005/123563 A1 (DORANZ BENJAMIN J [US] ET AL) 9 June 2005 (2005-06-09) * abstract * * paragraphs [0017] - [0021], [0026], [0027], [0176] - [0187], [0427] - [0430] * | 1-14 | |

TECHNICAL FIELDS SEARCHED (IPC)

G01N

The present search report has been drawn up for all claims

| Place of search | Date of completion of the search | Examiner |
|---|---|---|
| Munich | 11 September 2023 | Thumb, Werner |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 16 5274

This annex lists the patent family members relating to the patent documents cited in the above-mentioned European search report.
The members are as contained in the European Patent Office EDP file on
The European Patent Office is in no way liable for these particulars which are merely given for the purpose of information.

11-09-2023

| Patent document cited in search report | Publication date | Patent family member(s) | | Publication date |
|---|---|---|---|---|
| US 2005123563 A1 | 09-06-2005 | AU | 2004286197 A1 | 12-05-2005 |
| | | CA | 2534236 A1 | 12-05-2005 |
| | | EP | 1660638 A2 | 31-05-2006 |
| | | US | 2005123563 A1 | 09-06-2005 |
| | | US | 2014135230 A1 | 15-05-2014 |
| | | WO | 2005042695 A2 | 12-05-2005 |

EPO FORM P0459

For more details about this annex : see Official Journal of the European Patent Office, No. 12/82